# EUROPEAN PATENT APPLICATION

(11) **EP 4 201 364 A1**
(43) Date of publication of application: **28.06.2023**
(21) Application number: 21306915.6
(22) Date of filing: 23.12.2021
(51) Int. Cl.: A61B 90/13, A61B 34/10, A61B 90/00, A61B 34/20

(54) **METHOD FOR INDICATING AN INCISION TRAJECTORY BY A LASER OF AN INTRAOPERATIVE IMAGING SYSTEM**

(71) Applicant: Ecential Robotics, 38610 Gieres (FR); MinMaxMedical, 38400 Saint-Martin-d'Hères (FR)
(72) Inventor: LAVALLEE, Stéphane, 38610 GIERES (FR); LARUE, Jean-François, 38400 SAINT MARTIN D'HERES (FR); BEREAU, Marc, 38610 GIERES (FR); LEDIER, Vincent, 38610 GIERES (FR); ALMOSNINO, Denis, 38610 GIERES (FR); BELLY, Christian, 38610 GIERES (FR)
(74) Representative: Regimbeau

(57) **Abstract**

The invention relates to a method for indicating an incision trajectory, by an intraoperative imaging system (1) comprising a laser source (13), comprising the following steps:
- acquiring two X-ray images of a region of interest at different acquisition angles;
- designating, by a user, an element (33) on a displayed acquired image (32);
- computing, by a control unit, a target point (TP), based on a determined anatomical information of the patient (100) and on the designated element;
- indicating an incision trajectory to be followed to reach the target point (TP), by generating a laser beam (14) adapted to:
∘ mark the incision point (IP) on the patient's skin (110); and
∘ indicate the incision direction (ID) by an interaction between the laser beam (14) and an incision tool (20).

## Description

The present invention relates to a method for indicating an incision trajectory to be followed in a patient's body, performed by an intraoperative imaging system comprising a laser. The present invention also relates to an intraoperative imaging system comprising a laser, and adapted to indicate an incision trajectory to be followed in a patient's body.

### GENERAL TECHNICAL FIELD AND PRIOR ART

Intraoperative imaging systems are frequently used during medical procedures and surgical interventions, in order to acquire 2D or 3D X-ray images. The acquired images may be used to provide physicians with information about the anatomical situation of the patient and/or the position and orientation of surgical instruments during surgery.

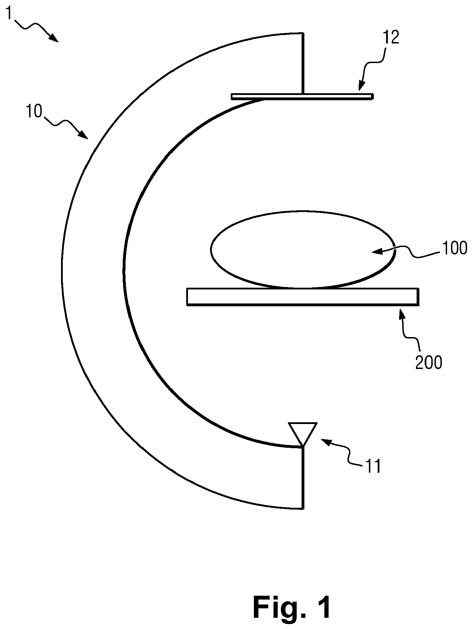

As illustrated in figure 1, an intraoperative imaging system 1 classically comprises a gantry 10, and an X-ray source 11 and X-ray detector 12 mounted on the gantry 10. In order to perform an X-ray imaging, a patient 100 is positioned on a table 200 between the X-ray source 11 and the X-ray detector 12.

The gantry is mounted on a base via an arm comprising mechanical connections which allow some degrees of freedom. This allows the gantry to be moved in different positions and orientations around and along the table on which the patient lies, so as to acquire the images required to perform the X-ray imaging. Movements of the gantry may include rotations and/or translations, performed simultaneously or successively.

The gantry can be a C-shaped gantry, the intraoperative imaging system being a C-arm, a U-shaped gantry, an O-shaped gantry, etc. Known C-arms that can produce 2D images are disclosed for example in documents US 7,927,014 B2, DE 10 2016 204 618 A1, US 2020/0121267 A1, and US 9,737,235 B2.

Intraoperative imaging systems may be used in the context of a surgical intervention carried out onto a patient's bone, for example for implantation of one or more objects in one or more bones. In order to implant the objects, the user performing the surgical procedure has to perform an incision in the patient, at a given incision point and with a given incision direction.

Document WO 2021/058087 A1 discloses a method comprising acquiring, by an intraoperative imaging system, images of a region of interest of a patient. The image is displayed on a display device, and a user sets a target in the displayed image. The method further comprises marking, via laser sources of the intraoperative imaging system, an incision point on the skin of the patient, below which the target may be found. The marking of the incision point is performed via control of the lasers of the imaging system. The surgeon may then place the tip of a tool on the marked incision point, so as to perform the incision in the patient.

However, WO 2021/058087 A1 merely indicates an incision point, but does not indicate any incision direction to be followed in order to reach the target. Therefore, the surgeon may perform the incision at the incision point, but at a wrong direction, which would cause the desired target not to be reached. Reaching a wrong target can be detrimental to the surgical procedure and can cause a risk to the patient.

GB 2 443 432 A discloses a method comprising acquiring, by an intraoperative imaging system, images of a region of interest of a patient. The method further comprises moving a mirror of the intraoperative system so as to reflect a laser beam produced by a laser source of the intraoperative system, such that the laser beam indicates the position of the axis joining the X-ray source and the X-ray detector of the X-ray imaging system. The method further comprises marking, via the laser beam, an incision point on the skin of the patient. The surgeon may then place the tip of a tool on the marked incision point. The method further comprises indicating an incision direction, corresponding to the orientation of the tip of the tool for which the laser beam impinges the free end of the tool.

However, the intraoperative system of GB 2 443 432 A, comprising the laser and mirrors, is complex. Furthermore, the marked incision point and the indicated incision direction do not take into account the anatomy of the patient, and thus lacks accuracy. Therefore, the surgeon may perform the incision at an imprecise incision point and/or incision direction, which would cause the desired target not to be reached. Instead, the surgeon could reach for example a space between bones rather than the bone itself. Reaching a wrong target can be detrimental to the surgical procedure and can cause a risk to the patient.

### GENERAL PRESENTATION OF THE INVENTION

An aim of the invention is to propose a simple and more accurate method for indicating an incision trajectory to be followed in a patient's body.

Another aim of the invention is to propose an intraoperative imaging system which allows to indicate simply and more accurately an incision trajectory to be followed in a patient's body.

According to a first aspect, the invention is directed towards a method for indicating an incision trajectory to be followed in a patient's body, the method being performed by an intraoperative imaging system comprising a laser source, wherein the method comprises the following steps:
- acquiring at least two 2D X-ray images of a region of interest of the patient's body at different acquisition angles using the intraoperative imaging system;
- displaying at least one of the acquired images on a display device;
- designating, by a user, an element on the displayed image;
- determining, by a control unit, an anatomical information of the patient based on the acquired images;
- computing, by the control unit, at least one target point in the region of interest, based on the determined anatomical information and on the designated element;
- determining, by the control unit, an incision trajectory to be followed in order to reach the target point, wherein the incision trajectory is defined by an incision point on the patient's skin and an incision direction; and
- controlling the intraoperative imaging system, by the control unit, so as to indicate the incision trajectory by generating by the laser source a laser beam adapted to:
   ∘ mark the incision point on the patient's skin; and
   ∘ indicate the incision direction by an interaction between the laser beam and an incision tool.

Some preferred but not limiting features of the method described above are the following, taken individually or in combination:
- indicating the incision direction further comprises the following steps, performed by the user:
   ∘ positioning a distal tip of the incision tool at the incision point;
   ∘ adjusting an orientation of the incision tool while keeping the distal tip at the incision point; and
   ∘ visually determining that the incision tool is oriented according to the incision direction, when the laser beam is in a predetermined interaction with the incision tool, for example when the laser beam is in a predetermined interaction with a proximal tip of the incision tool;
- the designated element corresponds to a projection on the displayed image of a spot of a bone or of a bone, the spot of the bone or the bone being located in the region of interest of the patient's body;
- the spot of the bone is a spot of a vertebra, and/or the bone is a vertebra;
- the method further comprises positioning a radiopaque marker at the incision point, wherein said step of positioning the radiopaque marker at the incision point is performed before the step of acquiring the at least two 2D X-ray images of the region of interest, wherein the method further comprises detecting the radiopaque marker in the at least two 2D X-ray acquired images, wherein the laser beam is adapted to mark the incision point at the radiopaque marker, and wherein the laser beam is adapted to indicate the incision direction corresponding to a direction of a straight line connecting the computed target point and said incision point;
- the method further comprises predetermining the incision direction, wherein the laser beam is adapted to mark the incision point corresponding to an intersection between the patient's skin and a straight line passing through the target point and oriented along the predetermined incision direction, and wherein the laser beam is adapted to indicate the predetermined incision direction;
- the computed target point corresponds to a point in a bone located in the region of interest of the patient's body at which a substantially point object is to be implanted in the bone, and said object comprises an electromagnetic transducer;
- several target points are computed, the laser beam is adapted to mark a unique incision point associated with the several target points, and the laser beam is adapted to successively indicate several incision directions respectively associated with the several target points;
- the computed target point corresponds to a point in a bone located in the region of interest of the patient's body at which a substantially segment object is to be positioned for subsequent implantation in the bone, and said object comprises a pin;
- the step of controlling the intraoperative imaging system comprises:
   ∘ a step of controlling a position and/or orientation of the at least one laser source; and/or
   ∘ a step of controlling a position and/or orientation of a gantry of the intraoperative imaging system;
- the step of controlling the position and/or orientation of the gantry comprises moving a motorized arm around at least one rotation axis of the motorized arm, the motorized arm includes at least three rotation axes and comprises a first end mounted to the gantry and a second end opposite to the first end and mounted to a mobile base, and said at least three rotation axes are directed along a substantially common vertical direction.
- the method further comprises moving the incision tool by a user so as to perform an incision along the incision trajectory.

According to a second aspect, the invention is direction towards an intraoperative imaging system adapted to indicate an incision trajectory to be followed in a patient's body, comprising:
- a gantry adapted to acquire at least two 2D X-ray images of a region of interest of the patient's body at different acquisition angles;
- a display device adapted to display at least one of the acquired images and to allow a user to designate an element on the displayed image; and
- a laser source adapted to generate a laser beam;

The intraoperative imaging system further comprises a control unit adapted to:
- determine an anatomical information of the patient based on the acquired images;
- compute at least one target point in the region of interest, based on the determined anatomical information and on the designated element;
- determine an incision trajectory to be followed in order to reach the target point, wherein the incision trajectory is defined by an incision point on the patient's skin and an incision direction;
- control the intraoperative imaging system so as to indicate the incision trajectory, by controlling the laser source so as to generate a laser beam adapted to:
   ∘ mark the incision point on the patient's skin; and
   ∘ indicate the incision direction by an interaction between the laser beam and an incision tool.

The gantry may be a C-shaped gantry.

The intraoperative imaging system may further comprise a motorized arm and a mobile base, wherein the motorized arm comprises a first end mounted to the gantry and a second end opposite to the first end and mounted to the mobile base.

The motorized arm may include at least three rotation axes, said at least three rotation axes are directed along a substantially common vertical direction.

### PRESENTATION OF THE FIGURES

Other features and advantages of the invention will emerge from the following description, which is purely illustrative and non-limiting and must be considered with respect to the appended figures in which:
- Figure 1, already commented, is a side section view of an intraoperative imaging system according to the prior art.
- Figure 2 is a side section view of an intraoperative imaging system according to the invention.
- Figure 3a is a section side section view of a patient having a bone in which an electromagnetic transducer is implanted.
- Figure 3b is a side section view of a patient having a bone in which a pin is implanted.
- Figure 4a is a side view section of a patient having a spine with three electromagnetic transducers implanted in three adjacent vertebrae.
- Figure 4b is a side section view of a patient having a spine with two pins implanted in two adjacent vertebrae.
- Figure 5 is a side section view of a patient illustrating a spine with three electromagnetic transducers implanted in three adjacent vertebrae by a way of a unique incision point.
- Figures 6a and 6b are side view sections of an intraoperative system adapted to indicate an incision trajectory to be followed in order to reach a target point located in a spine of a patient, wherein the incision trajectory is indicated by way of the method according to an embodiment of the invention, wherein the incision tool is respectively oriented in another direction than the incision direction and in the correct incision direction.
- Figure 7 is a perspective view of an intraoperative imaging system according to an embodiment of the invention.
- Figure 8 is a schematical view of a display device, and of a displayed image on which a user has designated an element, according to an embodiment of the invention.

### DESCRIPTION OF SEVERAL EMBODIMENTS

A method for indicating an incision trajectory to be followed in a patient's body is performed by an intraoperative imaging system 1 comprising a laser source 13. The method comprises the following steps:
- acquiring at least two 2D X-ray images of a region of interest of the patient's body at different acquisition angles using the intraoperative imaging system 1;
- displaying at least one of the acquired images on a display device 30;
- designating, by a user, an element 33 on the displayed image 32;
- determining, by a control unit, an anatomical information of the patient 100 based on the acquired images;
- computing, by the control unit, at least one target point TP in the region of interest, based on the determined anatomical information and on the designated element 33;
- determining, by the control unit, an incision trajectory to be followed in order to reach the target point TP, wherein the incision trajectory is defined by an incision point IP on the patient's skin 110 and an incision direction ID; and
- controlling the intraoperative imaging system 1, by the control unit, so as to indicate the incision trajectory by generating by the laser source 13 a laser beam 14 adapted to:
   ∘ mark the incision point IP on the patient's skin 110; and
   ∘ indicate the incision direction ID by an interaction between the laser beam 14 and an incision tool 20.

The steps of the method may be performed in any technically feasible order. For example, the acquisition of the at least two 2D X-ray images may be performed successively, or alternatively only one 2D X-ray image may be acquired and displayed for the user to designate the element, before the other 2D X-ray image is acquired. The determination of the anatomical information of the patient 100 may be performed before the display of the acquired image and/or before the designation of the element by the user.

The method allows to indicate both the incision point IP and the incision direction ID to the user. Thus, the user may accurately follow the incision trajectory with the incision tool 20, at the right incision point IP and with the right incision direction ID, in order to accurately follow the incision trajectory thus to accurately reach the target point TP.

Furthermore, the method takes into account an anatomical information of the patient 100 in order to compute the target point TP. Therefore, the incision trajectory is adapted to a given patient 100, instead of being a generic trajectory for all patient 100s. Consequently, the incision trajectory is determined more accurately, and thus allows the user to reach the target point TP with more accuracy, thereby improving the efficiency of the surgical procedure and reducing the risk for the patient 100 due to a wrong target point TP being reached.

The region of interest corresponds to a volume to be acquired by an X-ray imaging technique. More particularly, the region of interest may correspond to a bony structure of the patient 100. The region of interest may contain one or several vertebras to equip with one or several objects 50, 60.

The 2D X-ray images of the target region are acquired at at least two different acquisition angles.

For example, the intraoperative imaging system 1 may acquire a first 2D X-ray image with a nominal 0° alpha angle and a nominal 0° orbital angle, corresponding to a nominal position of the gantry 10, and then rotate the gantry 10 around at least one axis of rotation in order to acquire a second 2D X-ray image at a non-zero alpha angle and/or a non-zero orbital angle. Figure 1 illustrates a non-limiting example of an intraoperative imaging system in the nominal position.

The intraoperative imaging system 1 may acquire more than two 2D X-ray images, at more than two different acquisition angles. For example, some of the acquired images may present a non-zero alpha angle relative to the nominal position of the gantry 10, and/or some of the acquired images may present a non-zero orbital angle relative to the nominal position of the gantry 10. Thus, the precision of the determined anatomical information is improved.

The anatomical information of the patient 100 is adjusted in the two acquired image, in order to determine the anatomical information in the gantry's 10 reference frame.

The anatomical information may for example include:
- a position and/or orientation of one or more bones 120, such as vertebrae, in the gantry's 10 reference frame; and/or
- a distance between the patient's skin 110 and a bone 120, such as a vertebra, in which an object 50, 60 is to be implanted.

The display device 30 may include one or several screens 31. The display device 30 may display one, a selection of or all the acquired images. For example, if the user designates the element after only one 2D X-ray image has been acquired, the display device 30 displays the one acquired 2D X-ray image. If the user designates the element after all the 2D X-ray images have been acquired, the display device 30 may display one, a selection of or all the acquired 2D X-ray images. The user may choose one or more image(s), among the displayed images 32, on which to designate the element, for example may choose the displayed image 32 on which the element to designate is best visible.

The user may designate an element 33 on the displayed image 32 via a user interface which interacts with the display device 30.

One or several target point(s) TP is computed based on the determined anatomical information and on the one or several designated element(s) 33.

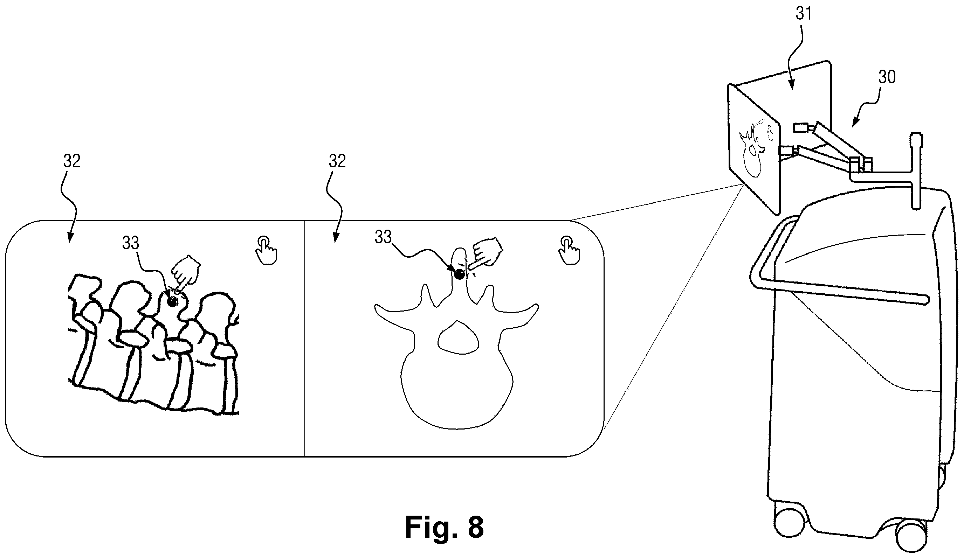

In a first embodiment, the designated element 33 corresponds to a projection on the displayed image 32 of a spot located in the region of interest of the patient's body, for example of a spot of a bone 120 located in the region of interest of the patient's body. The spot of the bone 120 may be a spot of a vertebra. The spot of the bone 120 may correspond to a spot at which an object 50, 60 is to be implanted or positioned for subsequent implantation in the bone 120. For example, the user may designate an element 33 corresponding to the projection on the displayed image 32 of a center of a vertebra 120 at which an object 50, 60 is to be implanted. The target point TP may then be computed so as to correspond to the center of the vertebra 120. Alternatively, the user may designate several elements corresponding to several projections on the displayed image 32 of several spots. Several target points TP are then computed so as to correspond to the several spots. For example, the user can designate three centers of three adjacent vertebras 120, at which three objects 50, 60 are to be implanted or positioned for subsequent implantation. Three target points TP are then computed so as to correspond to the three designated centers. The first embodiment is illustrated by way of a non-limiting example in figure 8, a same spot of a given vertebra 120, corresponding to a spot at which an object 50, 60 is to be implanted or positioned for subsequent implantation in the vertebra 120, being manually designated by the user on two different displayed images 32.

In a second embodiment, the designated element 33 corresponds to a projection on the displayed image 32 of a bone 120 located in the region of interest of the patient's body. The bone 120 may be a vertebra. Thus, the user designates the whole bone 120 of the patient 100 in the displayed image 32. The bone 120 may correspond to a bone 120 in which an object 50, 60 is to be implanted. For example, the user may designate the projection on the displayed image 32 of one or more vertebras 120 in which one or more objects 50, 60 are to be implanted. The user may also indicate a number of objects 50, 60 to implant in said one or more bones 120. By default, the number of objects 50, 60 to implant in a bone 120 may be set to one.

A number of target points TP corresponding to the indicated number of objects 50, 60 to be implanted in the bone(s) 120 may be computed. The computed target points TP correspond to the spots in the bone(s) 120 at which to implant the objects 50, 60. The target points TP are computed according to predetermined rules.

For example, if the user designates an element in the form of a vertebra 120, and specifies that two objects 50, 60 are to be implanted in the designated vertebra 120, two target points TP will be computed, respectively at one third and at two third of a length of the vertebra 120. Alternatively, if the user indicates three adjacent vertebras 120, and indicates that one object 50, 60 must be implanted in each of the designated vertebras 120, three target points TP will be computed by the control unit, each target point TP corresponding to a center of a designated vertebra 120.

The incision trajectory is defined by the incision point IP and the incision direction ID. The user following the incision trajectory thus moves the incision tool 20 in the patient's body substantially in a straight line oriented along the incision direction ID, from the incision point IP to the target point TP.

The intraoperative imaging system 1 may comprise for example one laser source 13, or two laser sources 13. Each laser source 13 is adapted to generated a laser beam 14. The incision point IP is marked on the patient's skin 110 by the laser beam 14.

When the intraoperative imaging system 1 comprises one laser source 13, the unique laser source 13 may be for example a point source, and the incision point IP corresponds to the point where the laser beam 14 hits the skin 110 of the patient 100. When the intraoperative imaging system 1 comprises two laser sources 13, the two laser sources 13 may be arranged and configured so that the two laser beam 14s together mark a cross on the skin 110 of the patient 100, so that the center of the cross corresponds to the incision point IP. In other words, each laser source 13 is configured to mark a segment on the patient's skin 110, the two laser sources 13 are offset by an angle and positioned so that the segments marked on the patient's skin 110 cross substantially at their center, said center corresponding to the incision point IP. The two laser sources 13 may be offset by a substantially 90° angle, so that the marked segments are perpendicular to each other.

The incision tool 20 may comprise a distal tip 22 and a proximal tip 21 opposite the distal tip 22. The incision tool 20 may be a needle.

Indicating the incision direction ID may further comprise the following steps, performed by the user:
- positioning a distal tip 22 of the incision tool 20 at the incision point IP;
- adjusting an orientation of the incision tool 20 while keeping the distal tip 22 at the incision point IP; and
- visually determining that the incision tool 20 is oriented according to the incision direction ID, when the laser beam 14 is in a predetermined interaction with the incision tool 20, for example when the laser beam 14 is in a predetermined interaction with a proximal tip 21 of the incision tool 20.

The predetermined interaction between the laser and the incision tool 20 may correspond to the laser beam 14 hitting the proximal tip 21 of the incision tool 20, the distal tip 22 of the incision tool 20 being positioned at the incision point IP, when the incision tool 20 is at the incision direction ID. Thus, the user may visually determine that the incision tool 20 is oriented along the incision direction ID. Indeed, the laser beam 14 is suddenly blocked by the proximal tip 21 of the incision tool 20, so may be visible on the proximal tip 21 of the tool and in any case does not mark the patient's skin 110 or at the distal tip 22 of the incision tool 20 anymore.

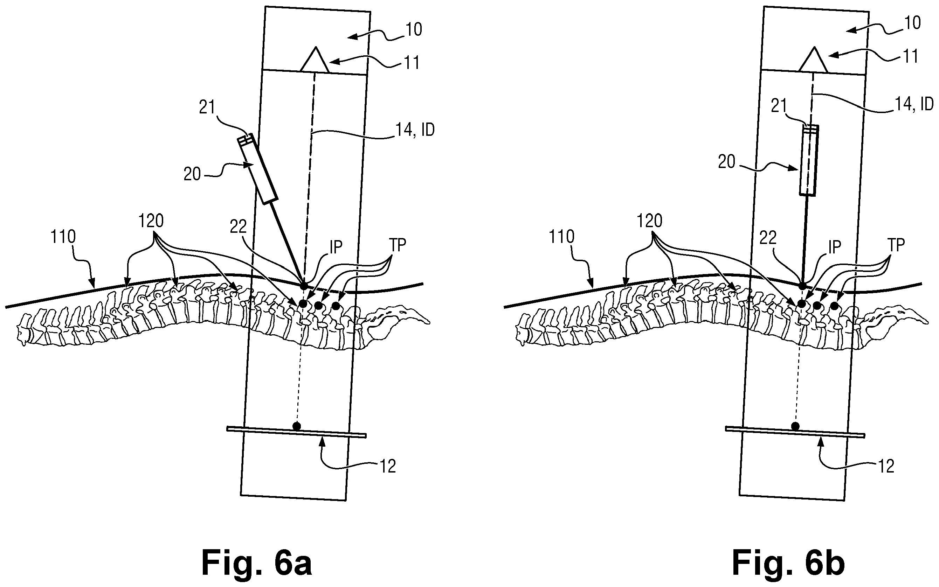

Figures 6a and 6b illustrate such an indication of the incision trajectory by interaction of the laser beam 14 with the incision tool 20. The laser beam 14 is oriented along the incision direction ID. The distal tip 22 of the incision tool 20 is positioned substantially at the marked incision point IP. In figure 6a, the incision tool 20 is oriented in another direction than the incision direction ID. Thus, the incision tool 20 does not interact with the laser beam 14, except at the incision point IP where the tip 22 of the incision tool 20 is positioned, and the laser beam 14 marks the incision point IP on the patient's skin 110 or on the tip 22 of the incision tool 20. In figure 6b, the incision tool 20 is oriented along the correct incision direction ID. Thus, the laser beam 14 hits the proximal tip 21 of the incision tool 20, thereby is impeded by the proximal tip 21 of the incision tool 20.

When the intraoperative imaging system 1 comprises at least two laser sources 13, one laser source 13 may be used to continuously mark the incision point IP and another laser source 13 may be used to indicate the incision direction ID. Alternatively, a unique laser source 13 may be used to first mark the incision point IP, and then to indicate the incision direction ID. The user positions the distal tip 22 of the incision tool 20 at the incision point IP, and then maintains the distal tip 22 at the incision point IP while modifying the orientation of the incision tool 20, thus does not need to continuously visualize said incision point IP.

Several objects 50, 60 may be implanted in one or several patient's bone(s) 120. Several incision trajectories are thus determined, each incision trajectory corresponding to a trajectory suitable for implantation of a respective object 50, 60 in a given bone 120. The several incision trajectories are indicated and followed successively by the user, the user following a first incision trajectory in order to implant a first object 50, 60, then a second incision trajectory in order to implant a second object 50, 60 in the same or a different bone 120, etc. Said several incision trajectories may be defined by a unique incision point IP and several incision directions ID, by several incision points IP and a unique incision direction ID, or by several incision points IP and several incision directions ID. Several incision points IP and/or incision directions ID are thus successively indicated.

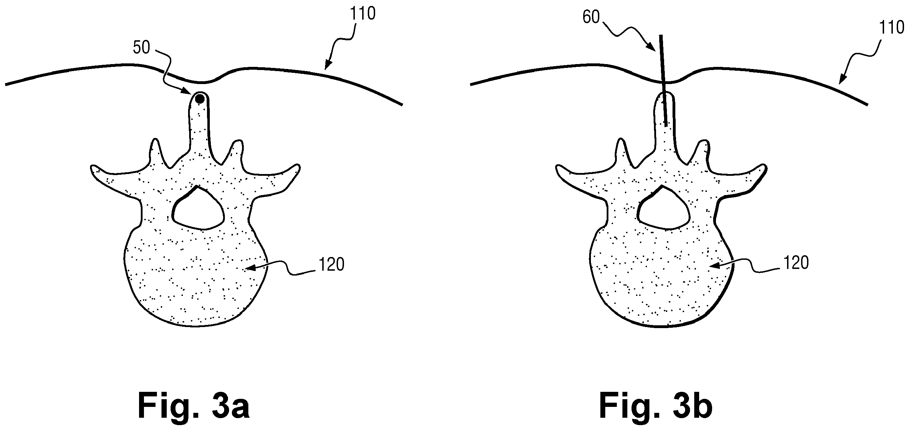

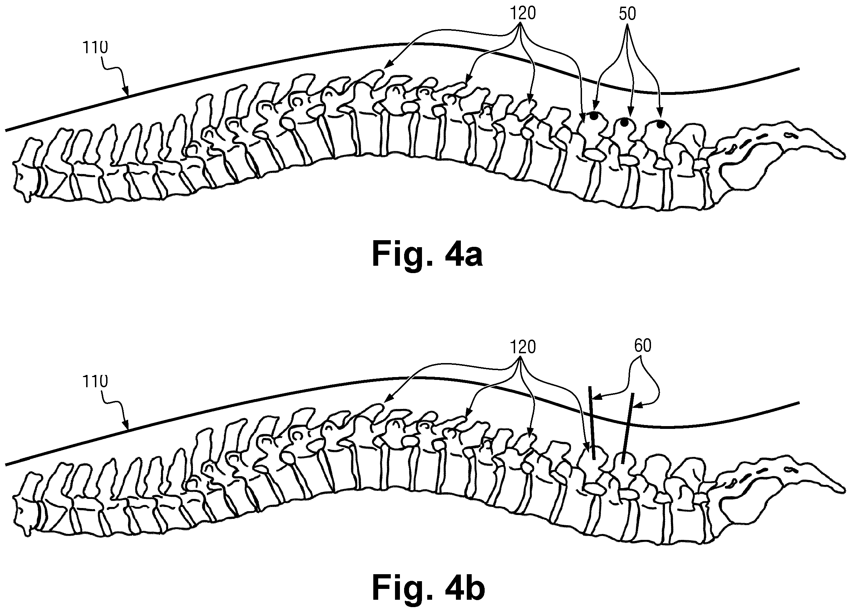

The object 50, 60 may be an electromagnetic transducer 50, also called electromagnetic sensor, as illustrated by way of a non-limiting example in figure 3a. An electromagnetic transducer 50 refers to a device adapted to either emit at least one electromagnetic field or receive such an electromagnetic field. The electromagnetic transducer 50 is a substantially point object 50, 60. The electromagnetic transducer 50 may be implanted at an implantation point of a patient's bone 120. The implantation point may correspond to a point located substantially on an external surface of the bone 120. The electromagnetic transducer 50 once implanted allows electromagnetic localization, instead of an optical localization, of a surgical tool whose tip is positioned proximate the electromagnetic transducer 50. Thus, the surgical tool can be accurately navigated in the patient 100 in order to perform the surgical procedure. Several electromagnetic transducers 50 may be implemented in one or several bones 120, for example in one or several vertebras, as illustrated by way of a non-limiting example in figure 4a. The precision of implantation of an electromagnetic transducer 50 should be of around 1-3 mm.

The object 50, 60 may be a pin 60, as illustrated by way of a non-limiting example in figure 3b. The pin 60 is substantially in the form of a segment extending along a certain length, delimited by a proximal tip and a distal tip opposite the proximal tip. The pin 60 must be inserted at least partially in the patient's bone 120, at an implantation point and along the incision direction ID. The implantation point is located substantially on an external surface of the bone 120. The distal tip of the pin 60 is to be positioned at the implantation point in order to then be inserted deeper in the bone 120 along the incision direction ID. When the pin 60 is inserted in the bone 120, the distal tip of the pin 60 is located inside the bone 120, while the proximal tip of the pin 60 is located outside the patient's body, the segment of the pin 60 passing through the incision point IP. Several pins 60 may be implemented in one or several bones 120, for example in several vertebras, as illustrated by way of a non-limiting example in figure 4b.

Several examples of realization for determining and indicating the incision trajectories are disclosed below.

In a first example of realization, the method further comprises positioning a radiopaque marker at the incision point IP, wherein said step of positioning the radiopaque marker at the incision point IP is performed before the step of acquiring the at least two 2D X-ray images of the region of interest. Thus, the radiopaque marker is visible in the acquired images. The radiopaque marker materializes the incision point IP. The method further comprises detecting the radiopaque marker in the at least two 2D X-ray acquired images.

The laser beam 14 is adapted to mark the incision point IP at the radiopaque marker. The laser beam 14 is adapted to indicate the incision direction ID corresponding to a direction of a straight line connecting the computed target point TP and said radiopaque marker located at the incision point IP. Thus, the incision trajectory is indicated based on the incision point IP given by the radiopaque marker, and on the incision direction ID defined by the radiopaque marker and the computed target point TP.

The first example of realization is particularly advantageous when the object 50, 60 to be implanted is an electromagnetic transducer 50.

In a second example of realization, the method further comprises predetermining the incision direction ID.

The laser beam 14 is adapted to mark the incision point IP corresponding to an intersection between the patient's skin 110 and a straight line passing through the target point TP and oriented along the predetermined incision direction ID. In other words, the straight line oriented along the predetermined incision direction ID and passing through the target point TP intersects with the skin 110 at the incision point IP. The laser beam 14 is also adapted to indicate the predetermined incision direction ID. Thus, the incision trajectory is determined based on the predetermined incision direction ID and on the target point TP.

The second example of realization is particularly advantageous when the object 50, 60 to be implanted is a pin 60. The predetermined incision direction ID corresponds to the desired orientation of the pin 60 once implanted in the bone 120. The target point TP corresponds to the implantation point at which the distal tip of the pin 60 should be positioned in order to be then implanted in the bone 120. That is to say, the user performs the incision by moving the incision tool 20 along the incision direction ID, and then performs the implantation of the pin 60 in the bone 120 without changing the orientation of the incision tool 20.

In a first implementation mode, the computed target point TP corresponds to a point in a bone 120 located in the region of interest of the patient's body at which a substantially point object 50, 60 is to be implanted in the bone 120. Said object 50, 60 may comprise an electromagnetic transducer 50.

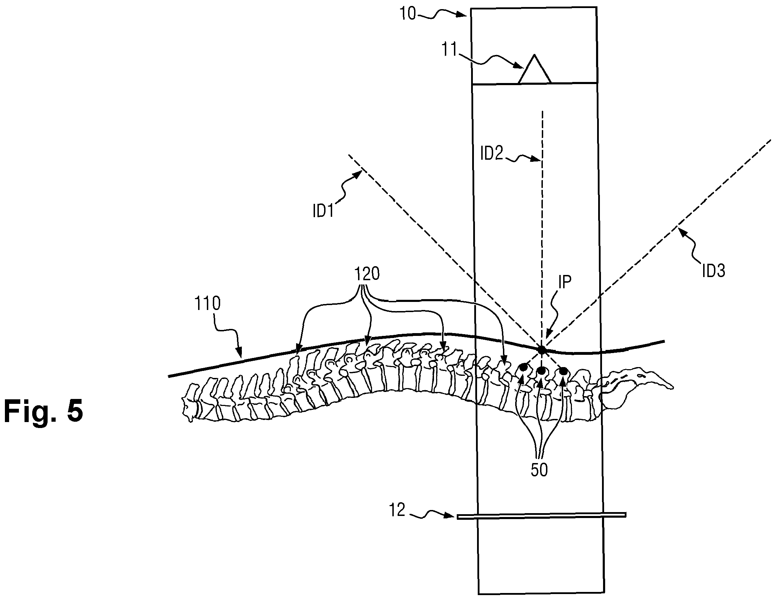

Several target points TP may be computed and the laser beam 14 may be adapted to mark a unique incision point IP associated with the several target points TP. The laser beam 14 is adapted to successively indicate several incision directions ID respectively associated with the several target points TP. Thus, a unique incision point IP allows to implant several electromagnetic transducers 50, which allows the surgical procedure to be less invasive. Several incision trajectories are defined by the unique incision point IP, and by several incision directions ID. As illustrated by way of a non-limiting example in figure 5, three target points TP may be computed, each target point TP corresponding to an implantation point of a respective electromagnetic transducer 50. Three incision directions ID1, ID2, ID3, each incision direction being associated with a target point TP, are defined, and one unique incision point IP is defined.

Alternatively, several target points TP may be computed and the laser beam 14 may be adapted to successively mark several incision points IP associated respectively with the several target points TP, and to indicate a unique incision direction ID associated with all the target points TP. That is to say, each electromagnetic transducer 50 to implant is associated with a respective incision trajectory defined by a respective incision point IP, and by an incision direction ID which is common to all the incision trajectories. The incision points IP are determined based on the unique incision direction ID and on the anatomical information. The unique incision direction ID may correspond to a direction substantially perpendicular to the skin 110 of the patient 100.

Alternatively, several target points TP may be computed, and the laser beam 14 may be adapted to successively mark several incision points IP associated respectively with the several target points TP, and to indicate several incision directions ID associated respectively with the several target points TP.

In a second implementation mode, the computed target point TP corresponds to a point in a bone 120 located in the region of interest of the patient's body at which a substantially segment object 50, 60 is to be positioned for subsequent implantation in the bone 120. Said object 50, 60 may comprise a pin 60.

The target point TP may correspond to the implantation point located substantially at the external surface of the bone 120, where the distal tip of the pin 60 is to be positioned in order to then be inserted in the bone 120 along the incision direction ID.

Several target points TP may be computed, and the laser beam 14 may be adapted to successively mark several incision points IP associated respectively with the several target points TP, and indicate a unique incision direction ID associated with all the target points TP. That is to say, each pin 60 to implant is associated with a respective incision trajectory defined by a respective incision point IP, and by an incision direction ID which is common to all the incision trajectories. The incision points IP are determined based on the incision direction ID and on the anatomical information. The unique incision direction ID may correspond to a direction substantially perpendicular to the skin 110 of the patient 100.

Alternatively, several target points TP may be computed, wherein the laser beam 14 is adapted to successively mark several incision points IP associated respectively with the several target points TP, and indicate several incision directions ID associated respectively with the several target points TP.

The step of controlling the intraoperative imaging system 1 may comprise:
- a step of controlling a position and/or orientation of the at least one laser source 13; and/or
- a step of controlling a position and/or orientation of a gantry 10 of the intraoperative imaging system 1.

More particularly, the position and/or orientation of the gantry 10 of the intraoperative system may be controlled while the at least one laser source 13 remains in a fixed position relative to the gantry 10.

The step of controlling the position and/or orientation of the gantry 10 may comprise moving a motorized arm 40 around at least one rotation axis of the motorized arm 40, wherein the motorized arm 40 includes at least three rotation axes and comprises a first end 41 mounted to the gantry 10 and a second end 42 opposite to the first end 41 and mounted to a mobile base 50, and wherein said at least three rotation axes are directed along a substantially common vertical direction.

When only one 2D X-ray image is acquired and then displayed for the user to designate the element, before the second 2D X-ray image is acquired, the method may further comprise the following steps:
- adjusting, based on the determined anatomical information, the designated element 33;
- displaying, on the display device 30, a second image including the adjusted designated element;
- confirming the adjusted designated element or designating a further adjusted designated element, by the user, in the displayed second image;
- updating the designated element, wherein the updated designated element 33 corresponds to the confirmed adjusted designated spot or to the further adjusted designated element; and
- computing the target, by the control unit, based on the determined anatomical information and on the updated designated element 33.

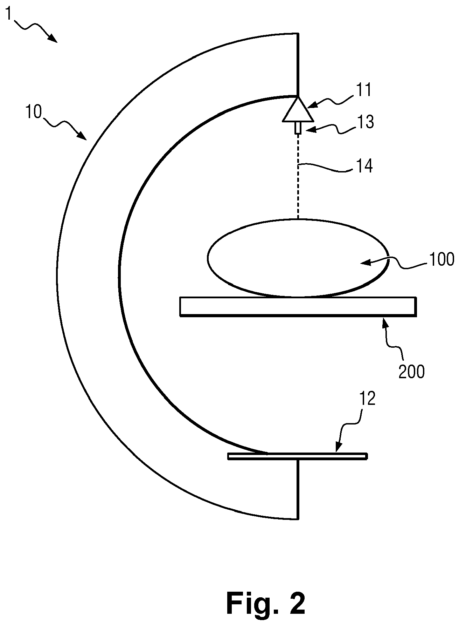

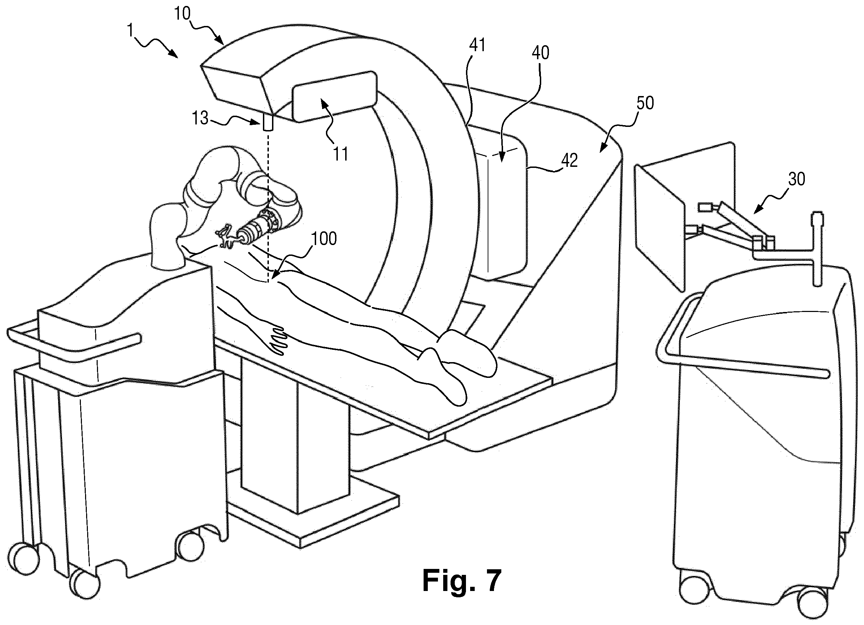

An intraoperative imaging system 1 adapted to indicate an incision trajectory to be followed in a patient's body, illustrated by way of a non-limiting example in figure 2 and figure 7, comprises:
- a gantry 10 adapted to acquire at least two 2D X-ray images of a region of interest of the patient's body at different acquisition angles;
- a display device 30 adapted to display at least one of the acquired images and to allow a user to designate an element 33 on the displayed image 32; and
- a laser source 13 adapted to generate a laser beam 14;
   wherein the intraoperative imaging system 1 further comprises a control unit adapted to:
- determine an anatomical information of the patient 100 based on the acquired images;
- compute at least one target point TP in the region of interest, based on the determined anatomical information and on the designated element 33;
- determine an incision trajectory to be followed in order to reach the target point TP, wherein the incision trajectory is defined by an incision point IP on the patient's skin 110 and an incision direction ID;
- control the intraoperative imaging system 1 so as to indicate the incision trajectory, by controlling the laser source 13 so as to generate a laser beam 14 adapted to:
   ∘ mark the incision point IP on the patient's skin 110; and
   ∘ indicate the incision direction ID by an interaction between the laser beam 14 and an incision tool 20.

The disclosed intraoperative imaging system 1 allows to indicate both the incision point IP and the incision direction ID to the user, while taking into account the anatomy of the patient 100. Thus, the user may accurately follow the incision trajectory with the incision tool 20, at the right incision point IP and along the right incision direction ID, and the incision trajectory is adapted to the patient 100. Therefore, the user may reach the target point TP as desired with an improved accuracy, thereby improving the efficiency of the surgical procedure and reducing the risk for the patient 100.

The intraoperative imaging system 1 further comprises an X-ray source 11 and an X-ray detector 12, mounted on the gantry 10. The laser source 13 may be mounted on the gantry 10. The laser beam 14 generated by the laser source 13 may be generated along a line passing through a center of the X-ray source 11 and the X-ray detector 12.

The X-ray source 11 and the X-ray detector 12 may be mounted on the gantry 10 so as to face each other, so that when the gantry 10 is positioned around a table 200 on which a patient 100 lies, the patient 100 is positioned substantially between the X-ray source 11 and the X-ray detector 12.

The gantry 10 may be substantially planar, that is to say may extend substantially in a gantry 10 plane. The gantry 10 may comprise an isocenter corresponding substantially to a center of a structure forming the gantry 10, the isocenter being comprised in the gantry 10 plane. The isocenter may substantially correspond to a middle of a segment connecting the X-ray source 11 and the X-ray detector 12.

The gantry 10 can be a C-shaped gantry 10, the intraoperative imaging system 1 being a C-arm, a U-shaped gantry 10, an O-shaped gantry 10, etc.

A C-shaped gantry 10 has a structure substantially shaped as a semicircle, ending with two opposite ends. Each of the X-ray source 11 and the X-ray detector 12 may be mounted next to a respective one of the two opposite ends of the C-shaped gantry 10. The isocenter of the C-shaped gantry 10 corresponds substantially to a center of the semicircular C-shaped structure.

A gantry 10 with an O-shaped structure may be similar to a gantry 10 with a C-shaped structure, except that the semicircle of the C-shaped structure is closed so as to form a full ring. The isocenter of the O-shaped gantry 10 may correspond substantially to a center of the circle of the O-shaped structure.

The intraoperative imaging system 1 may further comprise a motorized arm 40 and a mobile base 50. The control unit may be adapted to control an actuation of the mobile base 50 and/or the motorized arm 40 and/or the gantry 10.

A table 200 on which a patient 100 to be imaged lies may extend in a substantially horizontal plane. The horizontal plane is defined by a longitudinal direction, which corresponds to a direction of a length of the table 200, and a transverse direction, which is orthogonal to the longitudinal direction and corresponds to a direction of a width of the table 200. A ground of the room in which the X-ray imaging is performed may extend in the horizontal plane. A vertical direction corresponds to a direction perpendicular to the horizontal plane.

A rotation of the gantry 10 around the transverse rotation axis is characterized by an alpha angle. The alpha angle corresponds to an angle formed between the gantry 10 plane and a plane defined by the vertical direction and the transverse direction. In a nominal position of the gantry 10, the alpha angle is equal to 0°. When the alpha angle is not equal to zero, that is to say when the gantry 10 is rotated around the transverse rotation axis relative to the nominal position, the gantry 10 plane is inclined respective to the plane defined by the vertical direction and the transverse direction.

A rotation of the gantry 10 around an axis perpendicular to the gantry 10 plane and passing through the isocenter of the gantry 10 is called an orbital rotation. In a nominal position of the gantry 10, the orbital angle is equal to 0°.

The acquisition angles for acquiring the at least two 2D X-ray images may be obtained by rotating the gantry 10 of the intraoperative imaging system 1, so as to modify for example the alpha angle and/or the orbital angle. Controlling a position and/or orientation of a gantry 10 of the intraoperative imaging system 1 may include moving the gantry 10 in rotation(s) and/or translation(s), for example in rotation so as to modify the alpha angle and/or the orbital angle, in rotation around different axes of rotation, in translation along the longitudinal direction, the transverse direction and/or the vertical direction.

The control unit may comprise storage means, processing means such as a processor or microprocessor, and/or communication means.

The control unit may be integrated in the mobile base 50 of the intraoperative imaging system 1. Alternatively, the control unit may be integrated in a separate cart. Said separate cart may comprise a user interface. Alternatively, the control unit may be remote, for example may be placed in a separate control room of the hospital or in a data center.

The intraoperative imaging system 1 may further comprise a user interface adapted to allow a user to control a movement of the mobile base 50 and/or the motorized arm 40. The user interface may also be adapted to control an X-ray acquisition by the intraoperative imaging system 1.

The user interface may comprise switches, such as a power switch adapted to begin or stop an image acquisition, an emergency button adapted to stop a movement of the motorized arm 40 and/or the C-shaped gantry 10, etc.

The user interface may be integrated in the base, more particularly may be positioned on an external face of the base, so as to be visible by a user positioned near the base. Alternatively, the user interface may be remote from the base. For example, the user interface may be displayed on a screen of a computer distant from the base, the screen comprising virtual buttons allowing a user to control actuation of the motorized arm 40. Alternatively, the user interface may be duplicated remotely to allow remote control.

The motorized arm 40 of the intraoperative imaging system 1 may comprise a first end 41 mounted to the gantry 10, more specifically to the C-shaped gantry 10, and a second end 42 opposite to the first end 41 and mounted to the mobile base 50. Thus, the gantry 10 is mounted on the mobile base 50 by means of the motorized arm 40.

The motorized arm 40 may include at least three rotation axes. Said at least three rotation axes are directed along a substantially common vertical direction. The three rotation axes are thus substantially parallel and articulate different segments of the motorized arm 40 relative to each other, so as to allow the motorized arm 40 to access quickly a wide range of positions in a wide variety of directions. The motorized arm 40 may for example move the gantry 10 in translation in the horizontal plane.

The motorized arm 40 allows to move the gantry 10 along the several degrees of freedom, in rotation and/or in translation, which are necessary to perform the X-ray imaging and indicate the incision trajectory. The intraoperative imaging system 1 can be moved so as to follow complex trajectories, which enables imaging of a large variety of regions of interests, under a large variety of constraints.

The movement of the gantry 10 is managed by means of a single motorized arm 40, and is therefore simple. A motorized arm 40 comprising three substantially parallel rotation axes allows a high level of precision of movement, and therefore an accurate positioning and orienting of the gantry 10. Such an intraoperative imaging system 1 thus makes it possible to successively acquire several 2D X-ray images, by moving the gantry 10 before or between the successive acquisitions, while knowing that the movement command has been respected. Thus, the position of the X-ray source 11 and of the X-ray detector 12 are known for each acquisition of an imaging dataset.

The motorized arm 40 may be a robotic arm of the SCARA (Selective Compliance Articulated Robot Arm) type. The motorized arm 40 of the intraoperative imaging system 1 may be moved by electric motors.

Each rotation axis of the motorized arm 40 may correspond to a respective pivot connection. Such parallel-axis pivot connections provide a motorized arm 40 which is rigid in the vertical direction, but is slightly compliant in the other directions.

The motorized arm 40 may further present a translation axis in the vertical direction. The translation axis may coincide with one of the three rotation axes, and may be achieved by a slide connection. More particularly, at least one of the pivot connections of the motorized arm 40 may also be a slide connection. The motorized arm 40 is therefore a poly-articulated motorized arm 40, allowing translation of a segment of the motorized arm 40 in the vertical direction relative to another segment of the motorized arm 40.

The mobile base 50 may be moved according to the region of interest to acquire, for example may be moved so as to be positioned as close as possible to the region of interest to be acquired without hindering the user performing the X-ray imaging. This further reduces the complexity, weight and space needed by the intraoperative imaging system 1.

Other embodiments can be envisaged and a person skilled in the art can easily modify the embodiments or examples of realization exposed above or envisage others while remaining within the scope of the invention.

## Claims

1. Method for indicating an incision trajectory to be followed in a patient's body, the method being performed by an intraoperative imaging system (1) comprising a laser source (13), wherein the method comprises the following steps:
- acquiring at least two 2D X-ray images of a region of interest of the patient's body at different acquisition angles using the intraoperative imaging system (1);
- displaying at least one of the acquired images on a display device (30);
- designating, by a user, an element (33) on the displayed image (32);
- determining, by a control unit, an anatomical information of the patient (100) based on the acquired images;
- computing, by the control unit, at least one target point (TP) in the region of interest, based on the determined anatomical information and on the designated element (33);
- determining, by the control unit, an incision trajectory to be followed in order to reach the target point (TP), wherein the incision trajectory is defined by an incision point (IP) on the patient's skin (110) and an incision direction (ID); and
- controlling the intraoperative imaging system (1), by the control unit, so as to indicate the incision trajectory by generating by the laser source (13) a laser beam (14) adapted to:
∘ mark the incision point (IP) on the patient's skin (110); and
∘ indicate the incision direction (ID) by an interaction between the laser beam (14) and an incision tool (20).

2. Method according to claim 1, wherein indicating the incision direction (ID) further comprises the following steps, performed by the user:
- positioning a distal tip (22) of the incision tool (20) at the incision point (IP);
- adjusting an orientation of the incision tool (20) while keeping the distal tip (22) at the incision point (IP); and
- visually determining that the incision tool (20) is oriented according to the incision direction (ID), when the laser beam (14) is in a predetermined interaction with the incision tool (20), for example when the laser beam (14) is in a predetermined interaction with a proximal tip (21) of the incision tool (20).

3. Method according to claim 1 or claim 2, wherein the designated element (33) corresponds to a projection on the displayed image (32) of a spot of a bone (120) or of a bone (120), the spot of the bone (120) or the bone (120) being located in the region of interest of the patient's body.

4. Method according to claim 3, wherein the spot of the bone (120) is a spot of a vertebra, and/or wherein the bone (120) is a vertebra.

5. Method according to any one of claims 1 to 4, wherein the method further comprises positioning a radiopaque marker at the incision point (IP), wherein said step of positioning the radiopaque marker at the incision point (IP) is performed before the step of acquiring the at least two 2D X-ray images of the region of interest, wherein the method further comprises detecting the radiopaque marker in the at least two 2D X-ray acquired images, wherein the laser beam (14) is adapted to mark the incision point (IP) at the radiopaque marker, and wherein the laser beam (14) is adapted to indicate the incision direction (ID) corresponding to a direction of a straight line connecting the computed target point (TP) and said incision point (IP).

6. Method according to any one of claims 1 to 4, wherein the method further comprises predetermining the incision direction (ID), wherein the laser beam (14) is adapted to mark the incision point (IP) corresponding to an intersection between the patient's skin (110) and a straight line passing through the target point (TP) and oriented along the predetermined incision direction (ID), and wherein the laser beam (14) is adapted to indicate the predetermined incision direction (ID).

7. Method according to any one of claims 1 to 6, wherein the computed target point (TP) corresponds to a point in a bone (120) located in the region of interest of the patient's body at which a substantially point object (50, 60) is to be implanted in the bone (120), and wherein said object (50, 60) comprises an electromagnetic transducer (50).

8. Method according to claim 7, wherein several target points (TP) are computed, wherein the laser beam (14) is adapted to mark a unique incision point (IP) associated with the several target points (TP), and wherein the laser beam (14) is adapted to successively indicate several incision directions (ID) respectively associated with the several target points (TP).

9. Method according to any one of claims 1 to 6, wherein the computed target point (TP) corresponds to a point in a bone (120) located in the region of interest of the patient's body at which a substantially segment object (50, 60) is to be positioned for subsequent implantation in the bone (120), and wherein said object (50, 60) comprises a pin (60).

10. Method according to any one of claims 1 to 9, wherein the step of controlling the intraoperative imaging system (1) comprises:
- a step of controlling a position and/or orientation of the at least one laser source (13); and/or
- a step of controlling a position and/or orientation of a gantry (10) of the intraoperative imaging system (1).

11. Method according to claim 10, wherein the step of controlling the position and/or orientation of the gantry (10) comprises moving a motorized arm (40) around at least one rotation axis of the motorized arm (40), wherein the motorized arm (40) includes at least three rotation axes and comprises a first end (41) mounted to the gantry (10) and a second end (42) opposite to the first end (41) and mounted to a mobile base (50), and wherein said at least three rotation axes are directed along a substantially common vertical direction.

12. Intraoperative imaging system (1) adapted to indicate an incision trajectory to be followed in a patient's body, comprising:
- a gantry (10) adapted to acquire at least two 2D X-ray images of a region of interest of the patient's body at different acquisition angles;
- a display device (30) adapted to display at least one of the acquired images and to allow a user to designate an element (33) on the displayed image (32); and
- a laser source (13) adapted to generate a laser beam (14);
wherein the intraoperative imaging system (1) further comprises a control unit adapted to:
- determine an anatomical information of the patient (100) based on the acquired images;
- compute at least one target point (TP) in the region of interest, based on the determined anatomical information and on the designated element;
- determine an incision trajectory to be followed in order to reach the target point (TP), wherein the incision trajectory is defined by an incision point (IP) on the patient's skin (110) and an incision direction (ID);
- control the intraoperative imaging system (1) so as to indicate the incision trajectory, by controlling the laser source (13) so as to generate a laser beam (14) adapted to:
∘ mark the incision point (IP) on the patient's skin (110); and
∘ indicate the incision direction (ID) by an interaction between the laser beam (14) and an incision tool (20).

13. Intraoperative imaging system (1) according to claim 12, wherein the gantry (10) is a C-shaped gantry, wherein the intraoperative imaging system (1) further comprises a motorized arm (40) and a mobile base (50), wherein the motorized arm (40) comprises a first end (41) mounted to the C-shaped gantry (10) and a second end (42) opposite to the first end (41) and mounted to the mobile base (50), wherein the motorized arm (40) includes at least three rotation axes, said at least three rotation axes are directed along a substantially common vertical direction.
